# EUROPEAN PATENT SPECIFICATION

(11) **EP 1 297 822 B1**
(45) Date of publication and mention of the grant of the patent: **16.03.2005**
(21) Application number: 02020469.9
(22) Date of filing: 12.09.2002
(51) Int. Cl.: A61K 7/48

(54) **Cosmetic composition which comprises vitamin A or a derivative thereof**
Das Vitamin A oder dessen Derivat enthaltende kosmetische Zusammensetzung
Composition cosmétique comprenant de la vitamine A ou un dérivé de celle-ci

(30) Priority: 28.09.2001 JP 2001302348
(43) Date of publication of application: 02.04.2003
(73) Proprietor: Kosé Corporation, Chuo-ku Tokyo (JP)
(72) Inventor: Nakayama, Junko, Kosè Corporation, Res. & Dev.Div., Tokyo (JP)
(74) Representative: Albrecht, Thomas, Dr.

(56) References cited:
- EP-A- 0 421 333
- EP-A- 0 832 643
- EP-A- 0 858 799
- EP-A- 1 023 897
- WO-A-00/59456
- WO-A-01/70271
- WO-A-99/38485
- US-A- 5 788 954
- US-A- 5 891 470

## Description

This invention relates to cosmetics having excellent usability, feeling upon use and skin effect, in which vitamin A or a derivative thereof is stably formulated.

### BACKGROUND OF THE INVENTION

Vitamin A or its derivatives have been known as components effective for the prevention and treatment of diseases such as skin keratosis and also for the prevention of skin senescence, and have been formulated as active ingredients in various skin external preparations for these purposes.

However, the vitamin A or derivatives thereof are extremely unstable components by nature. That is, vitamin A or derivatives thereof are components which are easily deteriorated by reactions such as isomerization, oxidation degradation and hydrolysis induced by various causes such as light, air, heat and metal ions, so that it is apt to generate problems such as reduction of the substantial amount of effective components, and changes in color and odor.

Thus, it is the present situation that cosmetics prepared by formulating such vitamin A or derivatives thereof cannot avoid formulation of various stabilizing agents and limitation on the preparation of pharmaceuticals for ensuring their stability with the passage of time. Accordingly, their stability has been effected by formulating a large amount of oil, formulating an antioxidant and chelating agent and further formulating a water-soluble high polymer (JP-A-4-210902, JP-A-11-349439, JP-A-2001-122735).

However, since various chelating agents which are known to be effective for the stabilization of vitamin A or its derivatives are electrolytes, they exert a bad influence on the formulation of water-soluble high polymers as thickeners to cause problems such as aggregation and viscosity reduction in many cases, so that it is difficult to obtain sufficient thickening effect and satisfactory feeling upon use and skin effect. Also, formulation of a large amount of oil and formulation of a higher alcohol and a certain salt-resistant water-soluble high polymer are apt to generate bad influences in terms of the sense of touch such as insufficient aqueous feeling, heavy spreading and stickiness, and they are also unsatisfactory in terms of skin effect, such as skin elasticity after use.

In addition, even in the case of not formulating a chelating agent, stable formulation of vitamin A or its derivatives is not impossible but has a large limitation in preparing pharmaceuticals, so that it is difficult to obtain satisfactory feeling upon use and skin effect.

Formulations comprising vitamin A or a derivative thereof, an antioxidant and various polymers are also described in the following patent applications:
EP-A-0 832 643, US-A-5 788 954, WO 99/38485 A, WO 00/59456 A, EP-A-0 421 333, EP-A-0 858 799, EP-A-1 023 897, US-A-5 891 470, WO 01/70271 A.

### SUMMARY OF THE INVENTION

Accordingly, great concern has been directed toward the development of cosmetics in which vitamin A or its derivatives are stably formulated and which have excellent usability due to moderate viscosity, also have excellent feelings upon use such as aqueous feeling, rich and nourish feeling and no stickiness and can provide high skin effect.

Taking such situations into consideration, with the aim of solving these problems, the present inventors have conducted intensive studies and found as a result that a cosmetic composition in which vitamin A or its derivatives are stably formulated by the formulation of an alkali-soluble polymer emulsion thickener and an oil-soluble antioxidant and which has easily usable moderate viscosity, moderate rich and nourish feeling and feeling upon use during its use and high skin effect after its use can be obtained, and have accomplished the invention based on this finding.

Accordingly, the invention is a cosmetic composition which comprises, as essential components, vitamin A or a derivative thereof, an alkali-soluble polymer emulsion thickener and an oil-soluble antioxidant, wherein said alkali-soluble polymer emulsion thickener becomes transparent and thickens by the addition of an alkali agent, and the alkali-soluble polymer emulsion thickener is a copolymer of (A) at least one of acrylic acid and methacrylic acid, (B) one or two or more species selected from acrylic acid alkyl esters and methacrylic acid alkyl esters and (C) an ester of a polyoxyalkylene monoalkyl ether with acrylic acid or an ester of a polyoxyalkylene monoalkyl ether with methacrylic acid.

Further preferably, the cosmetic composition in which it contains a chelating agent.

### DETAILED DESCRIPTION OF THE INVENTION

Constitution of the present invention is described in the following.

The vitamin A or its derivatives to be used in the invention are formulated as components effective for the improvement of chapped skin, the prevention and treatment of diseases such as skin keratosis and also for the prevention of skin senescence.

Examples of the vitamin A or its derivatives to be used in the invention include vitamin A (retinol), vitamin A acetic acid ester, vitamin A palmitic acid ester, vitamin A propionic acid ester and astaxanthin, but they are not limited thereto and the whole vitamin A derivatives are included therein. Also, it is possible to use mixtures of these vitamin A or its derivatives, such as animal and plant oils containing them obtained from aquatic animals and plants. These may be used alone or as a combination of two or more as occasion demands.

Though formulating amount of the vitamin A or its derivatives to be used in the invention is not particularly limited, it is preferably 0.0001% by mass (the term "% by mass" have the same meaning with "% by weight"; to be referred simply to as "%" hereinafter) or more, more preferably from 0.001 to 10% based on the whole cosmetic composition.

The alkali-soluble polymer emulsion thickener to be used in the present invention is an essential component for stably formulating vitamin A or its derivatives, an oil-soluble antioxidant and a chelating agent, giving thickness and rich and nourish feeling at the time of use and also fresh feeling upon use, while giving moderate viscosity, and providing excellent skin effect, such as skin elasticity after use.

The alkali-soluble polymer emulsion thickener to be used in the invention is a low viscosity polymer aqueous dispersion by itself, which is a cloudy to slightly cloudy liquid called polymer emulsion. This agent increases viscosity and changes into transparent form by the addition of an alkali agent. The alkali-soluble polymer emulsion thickener to be used in the invention becomes transparent and thickens by the addition of an alkali agent as described above, and its illustrative examples include a polymer emulsion whose polymer moiety is a copolymer of acrylic acid, a methacrylic acid alkyl ester and an acrylic acid (polyoxyethylene monoalkyl ether) ester, and a polymer emulsion whose polymer moiety is a copolymer of acrylic acid, a methacrylic acid alkyl ester and a methacrylic acid (polyoxyethylene monoalkyl ether) ester, which may be used alone or as a combination of two or more in the present invention.

In order to obtain excellent usability and feeling upon use as the effects of the invention, the alkali-soluble polymer emulsion thickener is a polymer emulsion whose polymer moiety is a copolymer of the following three components:
(A) at least one of acrylic acid and methacrylic acid,
(B) one or two or more species selected from acrylic acid alkyl esters and methacrylic acid alkyl esters, and
(C) an ester of a polyoxyalkylene monoalkyl ether with acrylic acid or an ester of a polyoxyalkylene monoalkyl ether with methacrylic acid.

Examples of the copolymer obtained by copolymerizing these three components include ACRYLATES/CETETH-20 METHACRYLATE COPOLYMER, ACARYLATES/STEARETH-50 ACRYLATE COPOLYMER, ACRYLATES/STEARETH-20 METHACRYLATE COPOLYMER and ACRYLATES/BEHENETH-25 METHACRYLATE COPOLYMER described in ICID (International Cosmetic Ingredient Dictionary), and commercially available products such as Aculyn® 22 and Aculyn® 28 (both mfd. by Rohm & Haas) can be illustratively exemplified as the form of polymer emulsion and can be used suitably in the product of the invention.

The solid content of the alkali-soluble polymer emulsion thickener to be used in the invention is not particularly limited, but is preferably from 0.01 to 3% based on the whole cosmetic composition.

Also, the alkali-soluble polymer emulsion thickener can be thickened by adding an inorganic basic substance such as sodium hydroxide or potassium hydroxide or an organic basic substance such as triethanolamine, isopropanolamine or arginine.

The oil-soluble antioxidant to be used in the invention is formulated for the purpose of stabilizing vitamin A or its derivatives

. Though the oil-soluble antioxidant to be used in the invention is not particularly limited, its examples include dibutylhydroxytoluene (to be referred to as BHT hereinafter), butylhydroxyanisole (to be referred to as BHA hereinafter), α-, β-, γ- or δ-tocopherol. nordihydroguaiaretic acid, propyl gallate, oil-soluble vitamin C derivatives and sorbic acid, but it is possible to use one or two or more of other general oil-soluble components which have oxidation inhibitory effect and can be formulated in cosmetics.

Formulating amount of the oil-soluble antioxidant to be used in the invention is not particularly limited, but in order to obtain more higher oxidation degradation inhibitory effect of vitamin A or its derivatives, it is preferably 0.001% or more, more preferably 0.01% or more, based on the whole cosmetic composition. Though its upper formulating limit is not particularly limited, it is possible to formulate it roughly within the range of 10% or less based on the whole cosmetic composition.

According to the invention, it is possible to formulate a chelating agent for the purpose of further improving stability with the passage of time of vitamin A or its derivatives.

Though the chelating agent to be used in the invention is not particularly limited, its examples include alanine, edetic acid salts, sodium polyphosphate, sodium metaphosphate and phosphoric acid. These may be used alone or as a combination of two or more as occasion demands.

Formulating amount of the chelating agent to be used in the invention is not particularly limited, but in order to obtain more higher effect to improve the stability with the passage of time of vitamin A or its derivatives, it is preferably 0.001% or more, more preferably 0.01% or more, based on the whole cosmetic composition. Though its upper formulating limit is not particularly limited, it is possible to formulate it roughly within the range of 5.0% or less based on the whole cosmetic composition.

In addition to the essential components described in the foregoing, other components generally used in cosmetics, such as oily components including hydrocarbons, higher fatty acid esters, plant oil and fat, silicone oil and fluorine-containing oil, other high polymer substances than the above and aqueous components including alcohols, polyols and water, as well as a cosmetic powder, a surface active agent, an ultraviolet ray absorbing agent, a moisturizer, an antioxidant, a beauty component, a preservative, a perfume and a refrigerant, can be formulated in the cosmetics of the invention within such a range that they do not deteriorate the effect of the invention.

The cosmetics of the invention may be produced by utilizing the conventional preparation method. For example, the oily components and aqueous components are respectively dissolved and emulsified to prepare the cosmetics.

The following describes the invention further in detail with reference to examples.

Examples 1 to 6 and Comparative Examples 1 to 3: milky lotions

Milky lotions were prepared using the compositions shown in Table 1, by the preparation method described in the following. Usability (easily controllable moderate viscosity), feeling upon use (rich and nourish feeling at the time of use, aqueous feeling at the time of use), skin effect (skin elasticity after use) and stability with the passage of time of the thus obtained cosmetics were evaluated by the following method, with the results also shown in Table 1.

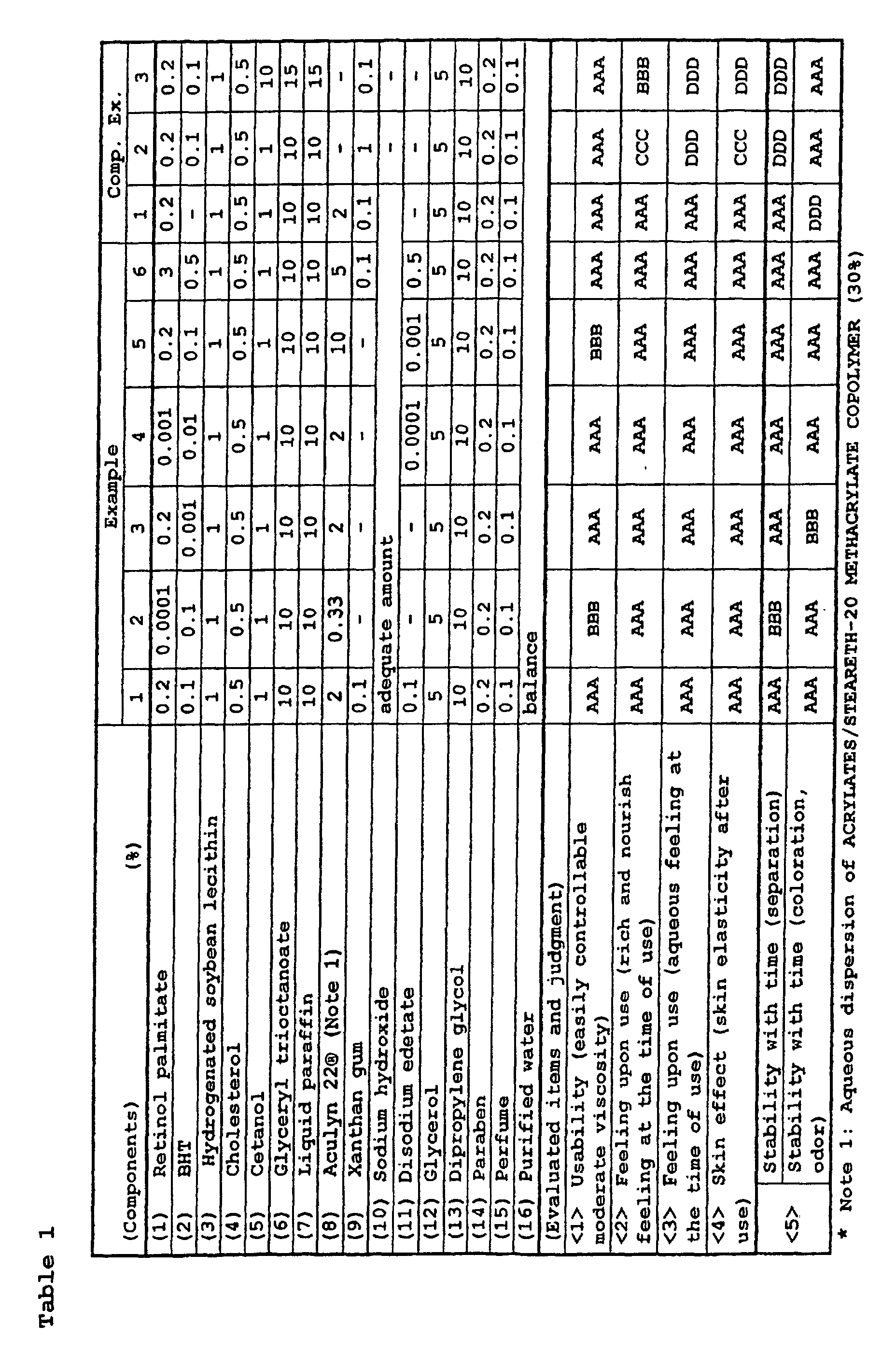

### (Preparation method)

A: Components (1) to (7) were dissolved by heating at 70°C.
B: Components (8) to (14) and component (16) were dissolved by heating at 70°C and then emulsified by adding the A.
C: The B was cooled to room temperature and then mixed with component (15) to obtain respective cosmetics.

### (Evaluation method: usability, feeling upon use, skin effect)

(1) Usability (easily controllable moderate viscosity), (2) feeling upon use (rich and nourish feeling at the time of use), (3) feeling upon use (aqueous feeling at the time of use) and (4) skin effect (skin elasticity after use) of respective samples of Examples 1 to 6 and Comparative Examples 1 to 3 were evaluated by ten expert panelists based on the following five step evaluation criteria (a), and the average value of evaluated points of each sample was judged based on the four step evaluation criteria (b).

### (a) Five step evaluation criteria

| (Evaluation point) | (Evaluation) |
|---|---|
| 4 | very good |
| 3 | good |
| 2 | fair |
| 1 | slightly bad |
| 0 | bad |

### (b) Four step evaluation criteria

| (Average value of evaluation points) | (Judgment) |
|---|---|
| 3.5 or more | AAA |
| 2.5 or more, less than 3.5 | BBB |
| 1.5 or more, less than 2.5 | CCC |
| less than 1.5 | DDD |

### (Evaluation method: stability with the passage of time)

Each sample was stored at 50°C for 1 month in a constant temperature chamber and judged from the viewpoints of (1) presence of separation in appearance and (2) changes in odor and presence of periodical coloration, by the following four step judging criteria (c) using conditions just after its preparation as the standard.

### (c) Four step judging criteria

| (Evaluation) | (Judgment) |
|---|---|
| No changes | AAA |
| Slight changes | BBB |
| Significant changes | CCC |
| Considerable changes | DDD |

As is evident from the results shown in Table 1, the Examples 1 to 6 according to the invention were cosmetics excellent in their usability, feeling upon use (rich and nourish feeling, aqueous feeling) and skin effect (skin elasticity after use). In addition, they were cosmetics excellent in stability with the passage of time too, because separation was not found and their odor and color were not changed even after 1 month of storage at 50°C in a constant temperature chamber.

### Example 7: Nourishing lotion

| | (Components) | (%) |
|---|---|---|
| (1) | Retinol propionate | 0.2 |
| (2) | Polyoxyethylene(60) hydrogenated castor oil | 1.0 |
| (3) | BHT | 0.1 |
| (4) | Glyceryl tri(capryl, caprylic acid) | 10 |
| (5) | Dipropylene glycol | 5 |
| (6) | Aculyn 22® (Note 1) | 5 |
| (7) | Alkyl modified carboxyvinyl polymer | 0.2 |
| (8) | Sodium hydroxide | adequate amount |
| (9) | Ethanol | 10 |
| (10) | N-acetyl-L-glutamic acid | 0.3 |
| (11) | Polyoxyethylene(10) methylglucoside | 2 |
| (12) | Disodium hydrogenphosphate | 0.3 |
| (13) | Preservative | adequate amount |
| (14) | Purified water | balance |

| | | |
|---|---|---|
| Note 1: Aqueous dispersion of ACRYLATES/STEARETH-20 METHACRYLATE COPOLYMER (30%) | | |

### (Preparation method)

A: Components (1) to (4) were uniformly mixed and dissolved.
B: Components (5) to (14) were uniformly mixed and dissolved.
C: A nourishing lotion was obtained by adding A to B and emulsifying the mixture.

The nourishing lotion obtained in Example 7 was excellent in usability, used feeling (rich and nourish feeling, aqueous feeling), skin effect (skin elasticity after use) and stability with the passage of time.

As has been described in the foregoing, the cosmetics of the invention are excellent in usability due to moderate viscosity and also excellent in feelings upon use such as rich and nourish feeling and aqueous feeling at the time of use and skin effect after use (skin elasticity after use) and have excellent stability with the passage of time.

While the invention has been described in detail and with reference to specific embodiments thereof, it will be apparent to one skilled in the art that various changes and modifications can be made therein without departing from the scope thereof.

## Claims

1. A cosmetic composition which comprises vitamin A or a derivative thereof, an alkali-soluble polymer emulsion thickener and an oil-soluble antioxidant, wherein said alkali-soluble polymer emulsion thickener becomes transparent and thickens by the addition of an alkali agent and wherein the alkali-soluble polymer emulsion thickener is a copolymer of (A), (B) and (C):
(A) at least one of acrylic acid and methacrylic acid,
(B) one or two or more species selected from acrylic acid alkyl esters and methacrylic acid alkyl esters, and
(C) an ester of a polyoxyalkylene monoalkyl ether with acrylic acid or an ester of a polyoxyalkylene monoalkyl ether with methacrylic acid.

2. The cosmetic composition according to claim 1, wherein it further comprises a chelating agent.

## Patentansprüche

1. Kosmetische Zusammensetzung, die Vitamin A oder ein Derivat davon, ein Alkali-lösliches Polymeremulsionsverdickungsmittel und ein Öl-lösliches Antioxidans umfasst, wobei das Alkali-lösliche Polymeremulsionsverdickungsmittel durch Zugabe eines alkalischen Mittels transparent wird und verdickt und wobei das Alkali-lösliche Polymeremulsionsverdickungsmittel ein Copolymer von (A), (B) und (C) ist:
(A) mindestens eines aus Acrylsäure und Methacrylsäure,
(B) ein oder zwei oder mehr Spezies, ausgewählt aus Acrylsäurealkylestern und Methacrylsäurealkylestern und
(C) ein Ester eines Polyoxyalkylenmonoalkylethers mit Acrylsäure oder ein Ester eines Polyoxyalkylenmonoalkylethers mit Methacrylsäure.

2. Kosmetische Zusammensetzung nach Anspruch 1, wobei sie des Weiteren ein chelatisierendes Mittel umfasst.

## Revendications

1. Composition cosmétique qui comprend de la vitamine A ou un dérivé de celle-ci, un épaississeur d'émulsion polymère soluble dans un alcali et un antioxydant soluble dans l'huile, dans laquelle ledit épaississeur d'émulsion polymère soluble dans un alcali devient transparent et épaissit par l'addition d'un agent d'alcali et dans laquelle l'épaississeur d'émulsion polymère soluble dans un alcali est un copolymère de (A), (B) et (C) :
(A) au moins un parmi l'acide acrylique et l'acide méthacrylique,
(B) une ou deux ou plusieurs espèces choisies parmi des esters alkyliques d'acide acrylique et des esters alkyliques d'acide méthacrylique, et
(C) un ester d'un monoalkyléther de polyoxyalkylène avec de l'acide acrylique ou un ester d'un monoalkyléther de polyoxyalkylène avec de l'acide méthacrylique.

2. Composition cosmétique selon la revendication 1, dans laquelle elle comprend en outre un agent chélatant.
